# EUROPEAN PATENT APPLICATION

(11) **EP 4 703 355 A1**
(43) Date of publication of application: **04.03.2026**
(21) Application number: 24796960.3
(22) Date of filing: 22.04.2024
(51) Int. Cl.: C07D 261/12, C07D 261/14, C07D 419/06, C07D 487/04

(54) **FLUORINE-CONTAINING ISOXAZOLONE COMPOUND AND PRODUCTION METHOD FOR SAME**

(30) Priority: 25.04.2023 JP 2023071550
(71) Applicant: Unimatec Co., Ltd., Tokyo 105-0012 (JP)
(72) Inventor: SEINO Junya, Kitaibaraki-shi, Ibaraki 319-1593 (JP); AOTSU Rie, Kitaibaraki-shi, Ibaraki 319-1593 (JP); TAKAHASHI Yusuke, Kitaibaraki-shi, Ibaraki 319-1593 (JP)
(74) Representative: Global IP Europe Patentanwaltskanzlei
(86) International application number: PCT/JP2024/015700
(87) International publication number: WO 2024/225214

(57) **Abstract**

To provide a 3-isoxazolone compound having a trifluoromethyl group at the 4-position and specific substituents at the 2- and 5-positions of the isoxazolone ring, and a 5-isoxazolone compound having a trifluoromethyl group at the 4-position and specific substituents at the 2- and 3-positions of the isoxazolone ring. To provide a production method capable of simply producing, without producing an O-substituted product as a by-product, a 3-isoxazolone compound and a 5-isoxazolone compound by reacting certain raw materials.

A fluorine-containing isoxazolone compound represented by the following general formula (1):

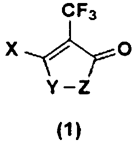

## Description

### Technical Field

The present invention relates to a fluorine-containing isoxazolone compound and a method for producing the same.

### Background Art

Compounds having an isoxazolone ring have been known to exhibit various pharmacological actions, among which 3- and 5-isoxazolone compounds having substituents at the 2-position have been used in the pharmaceutical and agrochemical fields. An example of a compound containing a 3-isoxazolone ring having a substituent at the 2-position includes Proxazolon, which is used as a growth inhibitor for turf. Examples of a compound containing a 5-isoxazolone ring having a substituent at the 2-position include Oxazonigelladine, which has an effect of enhancing the action of Acyclovir, and Parnafungin A1, which has polyadenosine polymerase inhibitory activity.

In this context, there has been growing interest in the introduction of substituents into the 4- and 5-positions of a 3-isoxazolone ring having a substituent at the 2-position, and the introduction of substituents into the 3- and 4-positions of a 5-isoxazolone ring having a substituent at the 2-position. Particularly, there has been growing interest in the development of methods for synthesizing a 5-isoxazolone compound having a trifluoromethyl group at the 4-position and specific substituents at the 2- and 3-positions, and a 3-isoxazolone compound having a trifluoromethyl group at the 4-position and specific substituents at the 2- and 5-positions.

As a method for producing 3-isoxazolone compound and 5-isoxazolone compound having substituents at the 2-position of the isoxazolone ring, a reaction for introducing substituents into the 2-positions of the isoxazolone rings of a 3-isoxazolone compound and a 5-isoxazolone compound can be employed. However, in attempting this kind of reaction, there are concerns that substituents might also be introduced onto oxygen atoms at the 3or 5-positions of the isoxazolone ring. More specifically, Non Patent Literature 1 and Non Patent Literature 2 report that in attempting to introduce a substituent into the 2-position of the isoxazolone ring of a 3-isoxazolone compound, a compound having the substituent introduced onto the oxygen atom at the 3-position of the isoxazolone ring is formed as a by-product. Besides, Non Patent Literature 3 and Non Patent Literature 4 report that in attempting to introduce a substituent into the 2-position of the isoxazolone ring of a 5-isoxazolone compound, a compound having the substituent introduced onto the oxygen atom at the 5-position of the isoxazolone ring is formed as a by-product.

### Document List

### Non Patent Literatures

Non Patent Literature 1: Bioorganic Chemistry, 2020, volume 100, page 103873
Non Patent Literature 2: Tetrahedron Letters, 2014, volume 55, pages 1693-1696
Non Patent Literature 3: ARKIVOC, 2001, volume 7, pages 88-103
Non Patent Literature 4: Journal Of Medicinal Chemistry, 1998, volume 41, pages 2513-2523

### Summary of Invention

### Technical Problem

It has been a concern that when a 3-isoxazolone compound and a 5-isoxazolone compound having a substituent at the 2-position of the isoxazolone ring are to be obtained by introducing substituents into the 2-position of a 3-isoxazolone compound and a 5-isoxazolone compound, the production, as byproducts, of a compound in which the substituent is introduced onto the oxygen at the 3-position in the isoxazolone ring in the case of the 3-isoxazolone compound or onto the oxygen at the 5-position in the isoxazolone ring in the case of the 5-isoxazolone compound (hereinafter referred to as an "O-substituted product") cannot be suppressed.

Thus, the present inventors have discovered that reacting certain raw materials enables a novel 3-isoxazolone compound having a trifluoromethyl group at the 4-position and specific substituents at the 2- and 5-positions of the isoxazolone ring, and a novel 5-isoxazolone compound having a trifluoromethyl group at the 4-position and specific substituents at the 2- and 3-positions of the isoxazolone ring to be obtained without producing an O-substituted product as a by-product, thereby completing the present invention. In other words, it is an object of the present invention to provide a 3-isoxazolone compound having a trifluoromethyl group at the 4-position and specific substituents at the 2- and 5-positions of the isoxazolone ring, and a 5-isoxazolone compound having a trifluoromethyl group at the 4-position and specific substituents at the 2- and 3-positions of the isoxazolone ring, which have not been known before. Besides, it is another object of the present invention to provide a production method capable of simply producing, without producing an O-substituted product as a by-product, a 3-isoxazolone compound having a trifluoromethyl group at the 4-position and specific substituents at the 2- and 5-positions of the isoxazolone ring, and a 5-isoxazolone compound having a trifluoromethyl group at the 4-position and specific substituents at the 2- and 3-positions of the isoxazolone ring by reacting certain raw materials.

### Solution to Problem

The gist configuration of the present invention is as follows:
[1] A fluorine-containing isoxazolone compound represented by the following general formula (1): 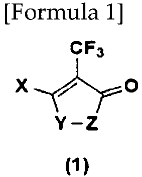 wherein
   X represents a fluorine atom, a heterocyclic group, -OA¹, -S(Oₙ)A¹, where n is an integer of 0 to 2, -O-NA¹A², -NA¹A², -NA³-NA¹A², or -N=A⁴,
   A¹, A², A³, and A⁴ each independently represent an organic group having 1 to 12 carbon atoms, and
      (i) Y is NR, where R is an alkyl group having 1 to 12 carbon atoms, an aryl group, an acyl group, an alkoxycarbonyl group, a cycloalkyl group, a carbamoyl group, or -(CH₂)ₐ-B¹, a is an integer of 1 to 12, and B¹ represents an organic group having 1 to 12 carbon atoms, and Z is O, or
      (ii) Y is O, and Z is NR, where R is an alkyl group having 1 to 12 carbon atoms, an aryl group, an acyl group, an alkoxycarbonyl group, a cycloalkyl group, a carbamoyl group, or -(CH₂)ₐ-B¹, a is an integer of 1 to 12, and B¹ represents an organic group having 1 to 12 carbon atoms.
[2] A method for producing a fluorine-containing isoxazolone compound, comprising a step of reacting a fluoroisobutanoic acid fluoride derivative represented by the following general formula (2) with a compound represented by the following general formula (7) or a salt thereof to obtain a fluorine-containing isoxazolone compound represented by the following general formula (1a): 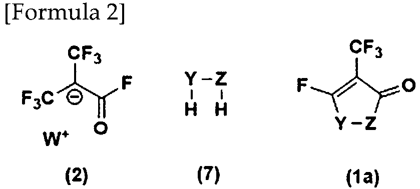 wherein
   (i) Y is NR, where R is an alkyl group having 1 to 12 carbon atoms, an aryl group, an acyl group, an alkoxycarbonyl group, a cycloalkyl group, a carbamoyl group, or -(CH₂)ₐ-B¹, a is an integer of 1 to 12, and B¹ represents an organic group having 1 to 12 carbon atoms, and Z is O, or
   (ii) Y is O, and Z is NR, where R is an alkyl group having 1 to 12 carbon atoms, an aryl group, an acyl group, an alkoxycarbonyl group, a cycloalkyl group, a carbamoyl group, or -(CH₂)ₐ-B¹, a is an integer of 1 to 12, and B¹ represents an organic group having 1 to 12 carbon atoms, and
   W⁺ represents an ammonium cation, an imidazolium cation, a pyridinium cation, or a phosphonium cation.
[3] A method for producing a fluorine-containing isoxazolone compound, comprising a step of reacting a fluoroisobutanoic acid fluoride derivative represented by the following general formula (2) with a compound represented by the following general formula (6) or a salt thereof and a compound represented by the following general formula (7) or a salt thereof to obtain a fluorine-containing isoxazolone compound represented by the following general formula (1b): 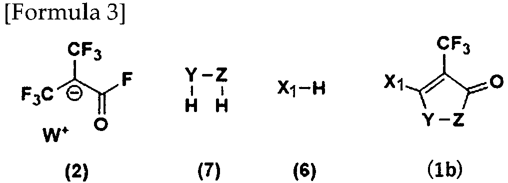 wherein
   X₁ represents a heterocyclic group, -OA¹, -S(Oₙ)A¹, where n is an integer of 0 to 2, -O-NA¹A², -NA¹A², -NA³-NA¹A², or -N=A⁴,
   A¹, A², A³, and A⁴ each independently represent an organic group having 1 to 12 carbon atoms,
      (i) Y is NR, where R is an alkyl group having 1 to 12 carbon atoms, an aryl group, an acyl group, an alkoxycarbonyl group, a cycloalkyl group, a carbamoyl group, or -(CH₂)ₐ-B¹, a is an integer of 1 to 12, and B¹ represents an organic group having 1 to 12 carbon atoms, and Z is O, or
      (ii) Y is O, and Z is NR, where R is an alkyl group having 1 to 12 carbon atoms, an aryl group, an acyl group, an alkoxycarbonyl group, a cycloalkyl group, a carbamoyl group, or -(CH₂)ₐ-B¹, a is an integer of 1 to 12, and B¹ represents an organic group having 1 to 12 carbon atoms, and
   W⁺ represents an ammonium cation, an imidazolium cation, a pyridinium cation, or a phosphonium cation.
[4] A method for producing a fluorine-containing isoxazolone compound, comprising a step of reacting a fluoroisobutene derivative represented by the following general formula (4) with a compound represented by the following general formula (7) or a salt thereof to obtain a fluorine-containing isoxazolone compound represented by the following general formula (1a): 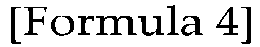 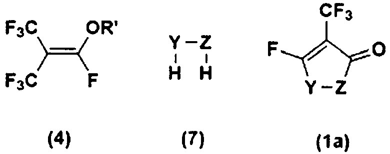 wherein
   R' represents a hydrocarbon group having 1 to 12 carbon atoms, and
   (i) Y is NR, where R is an alkyl group having 1 to 12 carbon atoms, an aryl group, an acyl group, an alkoxycarbonyl group, a cycloalkyl group, a carbamoyl group, or -(CH₂)ₐ-B¹, a is an integer of 1 to 12, and B¹ represents an organic group having 1 to 12 carbon atoms, and Z is O, or
   (ii) Y is O, and Z is NR, where R is an alkyl group having 1 to 12 carbon atoms, an aryl group, an acyl group, an alkoxycarbonyl group, a cycloalkyl group, a carbamoyl group, or -(CH₂)ₐ-B¹, a is an integer of 1 to 12, and B¹ represents an organic group having 1 to 12 carbon atoms.
[5] A method for producing a fluorine-containing isoxazolone compound, comprising a step of reacting a fluoroisobutene derivative represented by the following general formula (4) with a compound represented by the following general formula (6) or a salt thereof and a compound represented by the following general formula (7) or a salt thereof to obtain a fluorine-containing isoxazolone compound represented by the following general formula (1b): 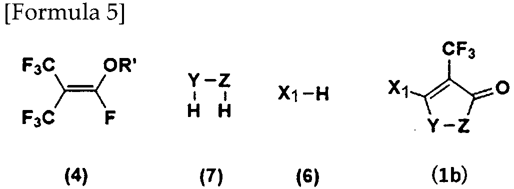 wherein
   R' represents a hydrocarbon group having 1 to 12 carbon atoms,
   X₁ represents a heterocyclic group, -OA¹, -S(Oₙ)A¹, where n is an integer of 0 to 2, -O-NA¹A², -NA¹A², -NA³-NA¹A², or -N=A⁴,
   A¹, A², A³, and A⁴ each independently represent an organic group having 1 to 12 carbon atoms, and
      (i) Y is NR, where R is an alkyl group having 1 to 12 carbon atoms, an aryl group, an acyl group, an alkoxycarbonyl group, a cycloalkyl group, a carbamoyl group, or -(CH₂)ₐ-B¹, a is an integer of 1 to 12, and B¹ represents an organic group having 1 to 12 carbon atoms, and Z is O, or
      (ii) Y is O, and Z is NR, where R is an alkyl group having 1 to 12 carbon atoms, an aryl group, an acyl group, an alkoxycarbonyl group, a cycloalkyl group, a carbamoyl group, or -(CH₂)ₐ-B¹, a is an integer of 1 to 12, and B¹ represents an organic group having 1 to 12 carbon atoms.
[6] A method for producing a fluorine-containing isoxazolone compound, comprising a step of reacting a fluoroisobutane derivative represented by the following general formula (5) with a compound represented by the following general formula (7) or a salt thereof to obtain a fluorine-containing isoxazolone compound represented by the following general formula (1a): 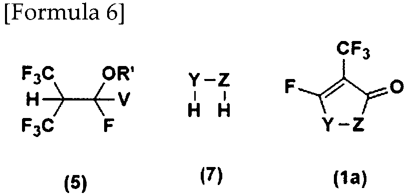 wherein
   R' represents a hydrocarbon group having 1 to 12 carbon atoms,
      (i) Y is NR, where R is an alkyl group having 1 to 12 carbon atoms, an aryl group, an acyl group, an alkoxycarbonyl group, a cycloalkyl group, a carbamoyl group, or -(CH₂)ₐ-B¹, a is an integer of 1 to 12, and B¹ represents an organic group having 1 to 12 carbon atoms, and Z is O, or
      (ii) Y is O, and Z is NR, where R is an alkyl group having 1 to 12 carbon atoms, an aryl group, an acyl group, an alkoxycarbonyl group, a cycloalkyl group, a carbamoyl group, or -(CH₂)ₐ-B¹, a is an integer of 1 to 12, and B¹ represents an organic group having 1 to 12 carbon atoms,
   V represents a halogen atom, -OA⁵, or -SOₘA⁵, where m is an integer of 0 to 2, and
   A⁵ represents an organic group having 1 to 12 carbon atoms.
[7] A method for producing a fluorine-containing isoxazolone compound, comprising a step of reacting a fluoroisobutane derivative represented by the following general formula (5) with a compound represented by the following general formula (6) or a salt thereof and a compound represented by the following general formula (7) or a salt thereof to obtain a fluorine-containing isoxazolone compound represented by the following general formula (1b): 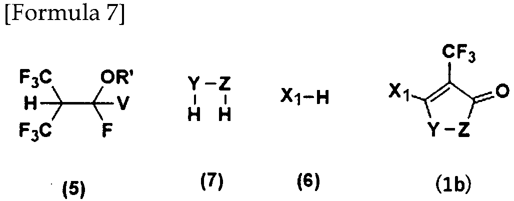 wherein
   R' represents a hydrocarbon group having 1 to 12 carbon atoms,
   X₁ represents a heterocyclic group, -OA¹, -S(Oₙ)A¹, where n is an integer of 0 to 2, -O-NA¹A², -NA¹A², -NA³-NA¹A², or -N=A⁴,
   A¹, A², A³, and A⁴ each independently represent an organic group having 1 to 12 carbon atoms,
      (i) Y is NR, where R is an alkyl group having 1 to 12 carbon atoms, an aryl group, an acyl group, an alkoxycarbonyl group, a cycloalkyl group, a carbamoyl group, or -(CH₂)ₐ-B¹, a is an integer of 1 to 12, and B¹ represents an organic group having 1 to 12 carbon atoms, and Z is O, or
      (ii) Y is O, and Z is NR, where R is an alkyl group having 1 to 12 carbon atoms, an aryl group, an acyl group, an alkoxycarbonyl group, a cycloalkyl group, a carbamoyl group, or -(CH₂)ₐ-B¹, a is an integer of 1 to 12, and B¹ represents an organic group having 1 to 12 carbon atoms,
   V represents a halogen atom, -OA⁵, or -SOₘA⁵, where m is an integer of 0 to 2, and
   A⁵ represents an organic group having 1 to 12 carbon atoms.

### Effects of Invention

It is possible to provide a 3-isoxazolone compound having a trifluoromethyl group at the 4-position and specific substituents at the 2- and 5-positions of the isoxazolone ring, and a 5-isoxazolone compound having a trifluoromethyl group at the 4-position and specific substituents at the 2- and 3-positions of the isoxazolone ring. Besides, it is possible to provide a production method capable of simply producing, without producing an O-substituted product as a by-product, a 3-isoxazolone compound having a trifluoromethyl group at the 4-position and specific substituents at the 2- and 5-positions of the isoxazolone ring, and a 5-isoxazolone compound having a trifluoromethyl group at the 4-position and specific substituents at the 2- and 3-positions of the isoxazolone ring by reacting certain raw materials.

### Description of Embodiments

### (Fluorine-containing Isoxazolone Compound)

The fluorine-containing isoxazolone compound of the present invention is represented by the following general formula (1): 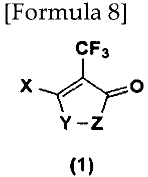 wherein
X represents a fluorine atom, a heterocyclic group, -OA¹, -S(Oₙ)A¹ (where n is an integer of 0 to 2), -O-NA¹A², -NA¹A², -NA³-NA¹A², or -N=A⁴,
A¹, A², A³, and A⁴ each independently represent an organic group having 1 to 12 carbon atoms, and
   (i) Y is NR (where R is an alkyl group having 1 to 12 carbon atoms, an aryl group, an acyl group, an alkoxycarbonyl group, a cycloalkyl group, a carbamoyl group, or -(CH₂)ₐ-B¹, a is an integer of 1 to 12, and B¹ represents an organic group having 1 to 12 carbon atoms), and Z is O, or
   (ii) Y is O, and Z is NR (where R is an alkyl group having 1 to 12 carbon atoms, an aryl group, an acyl group, an alkoxycarbonyl group, a cycloalkyl group, a carbamoyl group, or -(CH₂)ₐ-B¹, a is an integer of 1 to 12, and B¹ represents an organic group having 1 to 12 carbon atoms).

The fluorine-containing isoxazolone compound of the present invention has (a) specific substituents (-R, -CF₃, -X) on the 2-, 4-, and 5-positions of the 3-isoxazolone ring (in the case of (ii) above), and has (b) specific substituents (-R, -X, -CF₃) on the 2-, 3-, and 4-positions of the 5-isoxazolone ring (in the case of (i) above). Therefore, the compound can have an excellent effect from the viewpoint of structural expandability. In particular, desired biological activities (e.g., inhibitory activity against hormones or enzymes, fungicidal activity, insecticidal activity, and herbicidal activity) can be expected. In particular, examples of the fungicidal activity include fungicidal activity on bacteria that have a harmful effect on the human body and crops such as rice. In addition, since (a) the substituents at the 4- and 5-positions of the 3-isoxazolone ring are different groups (-CF₃ and -X) from each other, and (b) the substituents at the 3- and 4-positions of the 5-isoxazolone ring are different groups (-X and - CF₃) from each other, the compound can be easily derivatized into an asymmetric structure through elimination or reaction of these groups, and the compound can be expected to be used as an intermediate. More specifically, -CF₃ can be substituted by reacting the fluorine-containing isoxazolone compound to obtain a derivative. Moreover, -X can be modified by reacting the fluorine-containing isoxazolone compound under basic conditions to obtain a derivative. A fluorine-containing isoxazolone compound of one embodiment is useful in fields of electronic materials, such as organic semiconductors and liquid crystals.

The group X represents a fluorine atom, a heterocyclic group, -OA¹, - S(Oₙ)A¹ (where n is an integer of 0 to 2), -O-NA¹A², -NA¹A², -NA³-NA¹A², or - N=A⁴. A heterocyclic group used as the group X may have a monocyclic structure or a fused polycyclic structure as long as it is a heterocyclic group that includes one or more heteroatoms (oxygen atoms, sulfur atoms, or nitrogen atoms) as ring atoms. Furthermore, the heterocyclic group may further have a substituent, or may not have a substituent. Examples of the heterocyclic group include an isoxazolidinyl group, a thienyl group, a pyrrolyl group, a furyl group, a pyridyl group, a piperidinyl group, a quinolinyl group, an isoquinolinyl group, a pyrimidinyl group, a triazinyl group, a benzotriazole group, a methylindolyl group, an indolyl group, an isoindolyl group, a quinolyl group, an isoquinolyl group, a naphthyridyl group, a benzofuryl group, a pyrrolidyl group, an indolizinyl group, a benzothienyl group, an isobenzofuranyl group, a dibenzofuranyl group, a chromenyl group, an indazolyl group, a purinyl group, a pteridinyl group, a quinolizinyl group, a naphthyridinyl group, a quinoxalinyl group, a cinnolinyl group, a benzothiazolyl group, a benzisothiazolyl group, a quinazolinyl group, a phthalazinyl group, a benzoxazolyl group, a benzimidazolyl group, a pyridopyrimidinyl group, an isochromanyl group, a chromanyl group, an indolinyl group, an isoindolinyl group, a tetrahydroquinolyl group, a tetrahydroisoquinolyl group, a tetrahydroquinoxalinyl group, a dihydrophthalazinyl group, a carbazolyl group, a phenazinyl group, an acridinyl group, a phenanthridinyl group, a thianthrenyl group, a phenoxazinyl group, and a phenothiazinyl group. A¹, A², A³, and A⁴ each independently represent an organic group having 1 to 12 carbon atoms, and A¹, A², and A³ are monovalent groups, and A⁴ is a divalent group. A¹, A², A³, and A⁴ can be any organic group having 1 to 12 carbon atoms, and in addition to carbon atoms, may also contain a hydrogen atom, an oxygen atom, a sulfur atom, or a nitrogen atom. Examples of A¹, A², and A³ include a chain hydrocarbon group having 1 to 12 carbon atoms, an aromatic hydrocarbon group, an alicyclic hydrocarbon group, a heterocyclic group, -COA⁶, -C₁H₂₁-O-A⁷ (where 1 is an integer of 1 or more and 11 or less, A⁶ is an organic group having 1 to 11 carbon atoms, and A⁷ is an organic group having 1 to 12-1 carbon atoms), a -(CH₂)_{b}-NHCO-O-alkyl group (where b is an integer of 1 to 6), a -(CH₂)_{b}-NHCO-O-alkoxy group (where b is an integer of 1 to 6), a -(CH₂)_{b}-phenyl group (where b is an integer of 1 to 6), a -(CH₂)_{b}-alkoxy group (where b is an integer of 1 to 6), and a - (CH₂)_{b}-thienyl group (where b is an integer of 1 to 6). The chain hydrocarbon group may be a branched chain hydrocarbon group or an unbranched chain hydrocarbon group. The aromatic hydrocarbon group can be an aromatic hydrocarbon group having a substituent, or an aromatic hydrocarbon group not having a substituent. Besides, the aromatic hydrocarbon group may have a fused polycyclic structure. The alicyclic hydrocarbon group can be an alicyclic hydrocarbon group having a substituent, or an alicyclic hydrocarbon group not having a substituent. Besides, the alicyclic hydrocarbon group may have a bridged ring structure. The heterocyclic group used as A¹, A², and A³ can be a heterocyclic group that includes one or more heteroatoms (oxygen atoms, sulfur atoms, or nitrogen atoms) as ring atoms, and may have a monocyclic structure or a fused polycyclic structure. Besides, the heterocyclic group used as A¹, A², and A³ may have a substituent, or may not have a substituent. Examples of the heterocyclic group used as A¹, A², and A³ include a furyl group, a thienyl group, a pyrrolyl group, a pyranyl group, a pyridinyl group, an oxazolyl group, an isoxazolyl group, a thiazolyl group, an isothiazolyl group, an imidazolyl group, a pyrazolyl group, a pyrazinyl group, a pyridazinyl group, a benzofuranyl group, an isobenzofuranyl group, a pyrrolidinyl group, an indolyl group, an isoindolyl group, an indolizinyl group, a quinolyl group, an isoquinolyl group, a benzothienyl group, a chromenyl group, an indazolyl group, a purinyl group, a pteridinyl group, a cinnolinyl group, a pyrrolidinyl group, an imidazolidinyl group, a pyrazolidinyl group, a piperazolidinyl group, a piperidinyl group, a piperazinyl group, a morpholinyl group, and an imidazo[1,2-a]pyrimidinyl group. Examples of the heterocyclic group used as A⁴ include a cyclopentylidene group, a cyclohexylidene group, a diphenylmethylene group, a dibutylmethylene group, and -CB²B³ (where B² and B³ are each independently a hydrogen atom or an alkyl group having 1 to 6 carbon atoms). The group X is preferably a fluorine atom, a heterocyclic group, -OA¹, -O-NA¹A², or -NA¹A², more preferably a heterocyclic group, an alkoxy group, or -NA¹A², and still more preferably a heterocyclic group, an alkoxy group, or -NA¹A² (where A¹ and A² are each independently an alkyl group, an alkoxy group, an aryl group, an aryl group bonded to a divalent group such as -CH₂- (e.g., a -(CH₂)_{b}-phenyl group (where b is an integer of 1 to 6)), a heterocyclic group, a -(CH₂)_{b}-NHCO-O-alkyl group (where b is an integer of 1 to 6), a -(CH₂)_{b}-alkoxy group (where b is an integer of 1 to 6), or a -(CH₂)_{b}-thienyl group (where b is an integer of 1 to 6)). Besides, A¹, A², and A³ can each independently be an alkyl group, an alkoxy group, an aryl group, an aryl group bonded to a divalent group such as -CH₂-, a heterocyclic group, a -COA⁶ (where A⁶ is an organic group having 1 to 11 carbon atoms), or a -C₁H₂₁-O-A⁷, (where 1 is an integer of 1 or more and 11 or less, and A⁷ is an organic group having 1 to 12-l carbon atoms), and can also be a methoxy group, an ethoxy group, a propoxy group, a butoxy group, a methyl group, an ethyl group, a propyl group, a butyl group, a phenyl group, a benzyl group, or a heterocyclic group.

In the general formula (1),
(i) Y is NR (where R is an alkyl group having 1 to 12 carbon atoms, an aryl group, an acyl group (-COR¹; R¹ is an organic group), an alkoxycarbonyl group, a cycloalkyl group, a carbamoyl group, or -(CH₂)ₐ-B¹, a is an integer of 1 to 12, and B¹ represents an organic group having 1 to 12 carbon atoms), and Z is O, or
(ii) Y is O, and Z is NR (where R is an alkyl group having 1 to 12 carbon atoms, an aryl group, an acyl group (-COR¹; R¹ is an organic group), an alkoxycarbonyl group, a cycloalkyl group, a carbamoyl group, or -(CH₂)ₐ-B¹, a is an integer of 1 to 12, and B¹ represents an organic group having 1 to 12 carbon atoms).
Y in (i) above and Z in (ii) above are preferably -N(COOCₖH_{2K+1})- (where k is an integer of 1 or more), -N(CₖH₂ₖ₊₁)- (where k is an integer of 1 or more), or -N(CₖH₂ₖ₋₁)- (where CₖH₂ₖ₋₁ represents a cycloalkyl group, and k is an integer of 3 or more). Examples of B¹ in -(CH₂)ₐ-B¹ include a phenyl group, an alkoxy group, an alkoxyphenyl group, and a thienyl group. R is preferably -(CH₂)ₐ-B¹ (where a is an integer of 1 to 12, and B¹ represents an organic group having 1 to 12 carbon atoms), an alkoxycarbonyl group, an alkyl group, or a cycloalkyl group, more preferably -(CH₂)ₐ-B¹ (where a is an integer of 1 to 12, and B¹ represents an organic group having 1 to 12 carbon atoms), a methoxycarbonyl group, an ethoxycarbonyl group, a propoxycarbonyl group, a butoxycarbonyl group, a methyl group, an ethyl group, a propyl group, a butyl group, a cyclopentyl group, a cyclohexyl group, or a cycloheptyl group, and still more preferably -(CH₂)ₐ-B¹ (where a is an integer of 1 to 12, and B¹ represents an organic group having 1 to 12 carbon atoms), a methyl group, an isopropyl group, or a cyclohexyl group.

### (Method for Producing Fluorine-containing Isoxazolone Compound)

A method for producing a fluorine-containing isoxazolone compound of one embodiment includes:
(a) a step of reacting a fluoroisobutanoic acid fluoride derivative represented by the following general formula (2) with a compound represented by the following general formula (7) or a salt thereof to obtain a fluorine-containing isoxazolone compound represented by the following general formula (1a): 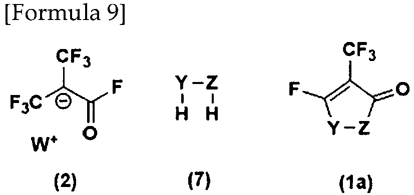 wherein
   (i) Y is NR (where R is an alkyl group having 1 to 12 carbon atoms, an aryl group, an acyl group, an alkoxycarbonyl group, a cycloalkyl group, a carbamoyl group, or -(CH₂)ₐ-B¹, a is an integer of 1 to 12, and B¹ represents an organic group having 1 to 12 carbon atoms), and Z is O, or
   (ii) Y is O, and Z is NR (where R is an alkyl group having 1 to 12 carbon atoms, an aryl group, an acyl group, an alkoxycarbonyl group, a cycloalkyl group, a carbamoyl group, or -(CH₂)ₐ-B¹, a is an integer of 1 to 12, and B¹ represents an organic group having 1 to 12 carbon atoms), and

   W⁺ represents an ammonium cation, an imidazolium cation, a pyridinium cation, or a phosphonium cation.
   R may be the same group as R above.

Examples of the ammonium cation include a triethylmethylammonium cation, a butyltrimethylammonium cation, and an N-methylquinuclidinium cation.

Examples of the imidazolium cation include a 1-butyl-3-methylimidazolium cation, a 1,3-dimethylimidazolium cation, and a 1-benzyl-3-methylimidazolium cation.

Examples of the pyridinium cation include a 1-methyl-4-dimethylaminopyridinium cation, a 1-methylpyridinium cation, and a 1,4-dimethylpyridinium cation.

Examples of the phosphonium cation include a tricyclohexylmethylphosphonium cation, a triphenylmethylphosphonium cation, and a tributylmethylphosphonium cation.

Examples of a salt of the compound represented by the general formula (7) include hydrochlorides, sulfates, and acetates.

Preferably, the step (a) of obtaining a fluorine-containing isoxazolone compound is preferably carried out in the presence of a fluoride ion scavenger. It is preferable to react a fluoroisobutanoic acid fluoride derivative represented by the general formula (2) with the compound represented by the general formula (7) or a salt thereof in the presence of a fluoride ion scavenger. The fluoride ion scavenger is not particularly limited as long as it is a substance having a function of capturing fluoride ions, and examples of the fluoride ion scavenger include lithium, sodium, magnesium, potassium, calcium, tetramethylammonium, trifluoroacetic acid, heptafluorobutyric acid, methanesulfonic acid, ethanesulfonic acid, benzenesulfonic acid, toluenesulfonic acid, trifluoromethanesulfonic acid, nonafluorobutanesulfonic acid, bis(trifluoromethanesulfonyl)imide, bis(nonafluorobutanesulfonyl)imide, N,N-hexafluoropropane-1,3-disulfonylimide, tetraphenylboric acid, tetrakis[3,5-bis(trifluoromethyl)phenyl]boric acid, and tetrakis(pentafluorophenyl)borate. It is considered that the cation derived from the fluoride ion scavenger captures fluoride ions liberated from the fluoroisobutanoic acid fluoride derivative represented by the general formula (2) during the reaction, and precipitates the fluoride ions as a salt having low solubility in an organic solvent to promote the reaction, and thus, the fluorine-containing isoxazolone compound represented by the general formula (1a) can be obtained in a high yield.

The reaction of (a) above, in which a fluoroisobutanoic acid fluoride derivative represented by the general formula (2) is reacted with a compound represented by the general formula (7) to obtain a fluorine-containing isoxazolone compound represented by the general formula (1a), is represented by the following reaction formula (A):

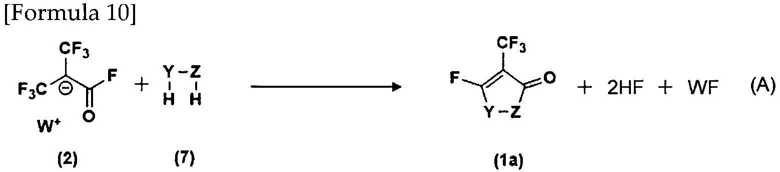

In the method for producing a fluorine-containing isoxazolone compound of this one embodiment, the reaction of (A) above can be carried out in a single step. Consequently, the fluorine-containing isoxazolone compound of the general formula (1a) can be obtained in a simplified manner. In the reaction of (a) above, the compound of the general formula (1a) is formed from the fluoroisobutanoic acid fluoride derivative represented by the general formula (2) and the compound represented by the general formula (7). Besides, -F and -CF₃ derived from the fluoroisobutanoic acid fluoride derivative are located at the 5-and 4-positions, respectively, of the 3-isoxazolone ring (in the case of (ii) above in which Y is O and Z is NR), and -F and -CF₃ derived from the fluoroisobutanoic acid fluoride derivative are located at the 3- and 4-positions, respectively, of the 5-isoxazolone ring (in the case of (i) above in which Y is NR and Z is O).

The reaction of (a) above is preferably carried out in the presence of a hydrogen halide scavenger. The hydrogen halide scavenger is a substance that has the function of capturing hydrogen fluoride (HF) formed from a hydrogen atom derived from the compound of the general formula (7) and a fluorine atom derived from the fluoroisobutanoic acid fluoride derivative of the general formula (2) in the reaction formula (A) above. As the hydrogen halide scavenger, sodium hydroxide, potassium hydroxide, calcium hydroxide, sodium carbonate, potassium carbonate, sodium fluoride, potassium fluoride, and organic nitrogen derivatives such as pyridine, triethylamine, diisopropylethylamine, diazabicyclononene, diazabicycloundecene, methyltriazabicyclodecene, diazabicyclooctane, and phosphazene base can be used.

A reaction temperature during the reaction of (a) above is preferably - 20 to 100°C, more preferably 0 to 50°C, and still more preferably 10 to 30°C. A reaction time during the reaction of (a) above is preferably 0.5 to 48 hours, more preferably 1 to 36 hours, and still more preferably 10 to 25 hours.

Examples of the solvent to be used in the reaction of (a) above include ethers such as tetrahydrofuran, diethyl ether, dioxane, monoglyme, diglyme, triglyme, and tetraglyme; aromatic hydrocarbons such as benzene, toluene, and xylene; a nitrile such as acetonitrile; and aprotic polar solvents such as dimethylformamide, dimethylacetamide, methylpyrrolidone, dimethylethyleneurea, tetramethylurea, dimethylsulfoxide, and sulfolane.

A method for producing a fluorine-containing isoxazolone compound of another embodiment includes:
(b) a step of reacting a fluoroisobutanoic acid fluoride derivative represented by the following general formula (2) with a compound represented by the following general formula (6) or a salt thereof and a compound represented by the following general formula (7) or a salt thereof to obtain a fluorine-containing isoxazolone compound represented by the following general formula (1b): 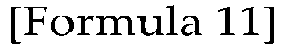 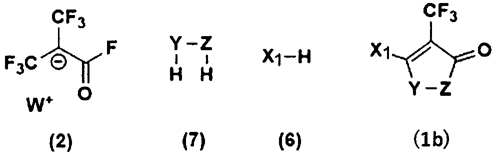 wherein
X₁ represents a heterocyclic group, -OA¹, -S(Oₙ)A¹ (where n is an integer of 0 to 2), -O-NA¹A², -NA¹A², -NA³-NA¹A², or -N=A⁴,
A¹, A², A³, and A⁴ each independently represent an organic group having 1 to 12 carbon atoms,
   (i) Y is NR (where R is an alkyl group having 1 to 12 carbon atoms, an aryl group, an acyl group, an alkoxycarbonyl group, a cycloalkyl group, a carbamoyl group, or -(CH₂)ₐ-B¹, a is an integer of 1 to 12, and B¹ represents an organic group having 1 to 12 carbon atoms), and Z is O, or
   (ii) Y is O, and Z is NR (where R is an alkyl group having 1 to 12 carbon atoms, an aryl group, an acyl group, an alkoxycarbonyl group, a cycloalkyl group, a carbamoyl group, or -(CH₂)ₐ-B¹, a is an integer of 1 to 12, and B¹ represents an organic group having 1 to 12 carbon atoms), and
W⁺ represents an ammonium cation, an imidazolium cation, a pyridinium cation, or a phosphonium cation.
A¹, A², A³, A⁴, B¹, R, and W⁺ may be the same groups respectively as A¹, A², A³, A⁴, B¹, R, and W⁺ above.

Examples of a salt of the compound represented by the general formula (6) include hydrochlorides, sulfates, and acetates.

Examples of a salt of the compound represented by the general formula (7) include hydrochlorides, sulfates, and acetates.

Preferably, the step (b) of obtaining a fluorine-containing isoxazolone compound is preferably carried out in the presence of a fluoride ion scavenger. It is preferable to react the fluoroisobutanoic acid fluoride derivative represented by the general formula (2) with the compound represented by the general formula (6) or a salt thereof and the compound represented by the general formula (7) or a salt thereof in the presence of a fluoride ion scavenger. The fluoride ion scavenger is not particularly limited as long as it is a substance having a function of capturing fluoride ions, and examples thereof include the same fluoride ion scavengers to be used in the step (a). It is considered that the cation derived from the fluoride ion scavenger captures fluoride ions liberated from the fluoroisobutanoic acid fluoride derivative represented by the general formula (2) during the reaction, and precipitates the fluoride ions as a salt having low solubility in an organic solvent to promote the reaction, and thus, the fluorine-containing isoxazolone compound represented by the general formula (1b) can be obtained in a high yield.

The reaction of (b) above between a fluoroisobutanoic acid fluoride derivative represented by the general formula (2) and compounds represented by the general formulae (6) and (7) is represented by the following reaction formula (B):

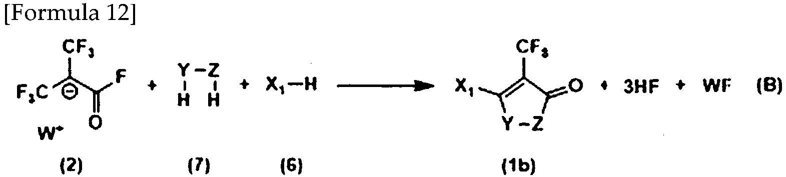

In the method for producing a fluorine-containing isoxazolone compound of this another embodiment, the reaction of (B) above can be carried out in a single step. Consequently, the fluorine-containing isoxazolone compound of the general formula (1b) can be obtained in a simplified manner. In the reaction of (b) above, a cyclic isoxazolone ring structure is formed between the fluoroisobutanoic acid fluoride derivative represented by the general formula (2) and the compound of the general formula (7). Besides, -X₁ and -CF₃ derived from the compound of the general formula (6) and the fluoroisobutanoic acid fluoride derivative are located at the 5- and 4-positions, respectively, of the 3-isoxazolone ring (in the case of (ii) above in which Y is O and Z is NR), and -X₁ and -CF₃ derived from the compound of the general formula (6) and the fluoroisobutanoic acid fluoride derivative are located at the 3- and 4-positions, respectively, of the 5-isoxazolone ring (in the case of (i) above in which Y is NR and Z is O).

A reaction temperature during the reaction of (b) above is preferably - 20 to 100°C, more preferably 0 to 50°C, and still more preferably 10 to 30°C. A reaction time during the reaction of (b) above is preferably 0.5 to 48 hours, more preferably 1 to 36 hours, and still more preferably 10 to 25 hours. In the reaction of (b) above, the same hydrogen halide scavenger and solvent as in (a) above can be used.

A method for producing a fluorine-containing isoxazolone compound of another embodiment includes:
(c) a step of reacting a fluoroisobutene derivative represented by the following general formula (4) with a compound represented by the following general formula (7) or a salt thereof to obtain a fluorine-containing isoxazolone compound represented by the following general formula (1a): 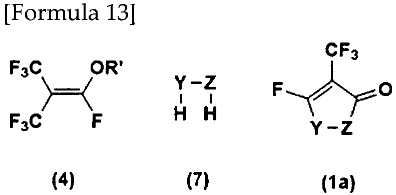 wherein
R' represents a hydrocarbon group having 1 to 12 carbon atoms,
   (i) Y is NR (where R is an alkyl group having 1 to 12 carbon atoms, an aryl group, an acyl group, an alkoxycarbonyl group, a cycloalkyl group, a carbamoyl group, or -(CH₂)ₐ-B¹, a is an integer of 1 to 12, and B¹ represents an organic group having 1 to 12 carbon atoms), and Z is O, or
   (ii) Y is O, and Z is NR (where R is an alkyl group having 1 to 12 carbon atoms, an aryl group, an acyl group, an alkoxycarbonyl group, a cycloalkyl group, a carbamoyl group, or -(CH₂)ₐ-B¹, a is an integer of 1 to 12, and B¹ represents an organic group having 1 to 12 carbon atoms).
R may be the same group as R above.

Examples of R' include an alkyl group, more specifically a methyl group and an ethyl group. Examples of a salt of the compound represented by the general formula (7) include hydrochlorides, sulfates, and acetates.

The step (c) of obtaining a fluorine-containing isoxazolone compound is preferably carried out in the presence of a nucleophilic reagent. Examples of the nucleophilic reagent include 1-methylimidazole, 4-dimethylaminopyridine, 1,4-diazabicyclo[2.2.2]octane, and triphenylphosphine. The use of the nucleophilic reagent can effectively eliminate the substituent -R' from the fluoroisobutene derivative represented by the general formula (4) to promote the generation of the fluorine-containing isoxazolone compound represented by the general formula (1a). The step (c) of obtaining a fluorine-containing isoxazolone compound is preferably carried out in the presence of a fluoride ion scavenger. It is preferable to react the fluoroisobutene derivative represented by the general formula (4) with the compound represented by the general formula (7) or a salt thereof in the presence of a fluoride ion scavenger. The fluoride ion scavenger is not particularly limited as long as it is a substance having a function of capturing fluoride ions, and examples thereof include the same fluoride ion scavengers to be used in the step (a) above. It is considered that the cation derived from the fluoride ion scavenger captures fluoride ions liberated from the fluoroisobutane derivative represented by the general formula (4) during the reaction, and precipitates the fluoride ions as a salt having low solubility in an organic solvent to promote the reaction, and thus, the fluorine-containing isoxazolone compound represented by the general formula (1a) can be obtained in a high yield.

The reaction of (c) above, performed in the presence of a nucleophilic reagent, 1-methylimidazole, between the fluoroisobutene derivative represented by the general formula (4) and the compound represented by the general formula (7), is represented by the following reaction formula (C):

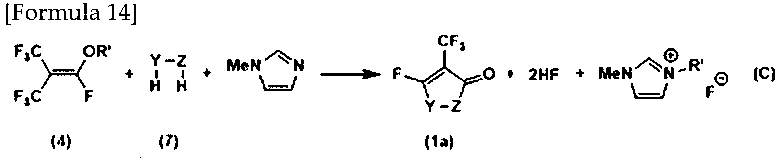

In the method for producing a fluorine-containing isoxazolone compound of this another embodiment, the reaction of (C) above can be carried out in a single step. Consequently, the fluorine-containing isoxazolone compound of the general formula (1a) can be obtained in a simplified manner. In the reaction of (c) above, a cyclic isoxazolone ring structure is formed between the fluoroisobutene derivative of the general formula (4) and Y and Z derived from the compound of the general formula (7). Besides, -F and -CF₃ derived from the fluoroisobutene derivative are located at the 5- and 4-positions, respectively, of the 3-isoxazolone ring (in the case of (ii) above in which Y is O and Z is NR), and -F and -CF₃ derived from the fluoroisobutene derivative are located at the 3-and 4-positions, respectively, of the 5-isoxazolone ring (in the case of (i) above in which Y is NR and Z is O).

A reaction temperature during the reaction of (c) above is preferably - 20 to 100°C, more preferably 0 to 50°C, and still more preferably 10 to 30°C. A reaction time during the reaction of (c) above is preferably 0.5 to 48 hours, more preferably 1 to 36 hours, and still more preferably 10 to 25 hours. In the reaction of (c) above, the same hydrogen halide scavenger and solvent as in (a) above can be used.

A method for producing a fluorine-containing isoxazolone compound of another embodiment includes:
(d) a step of reacting a fluoroisobutene derivative represented by the following general formula (4) with a compound represented by the following general formula (6) or a salt thereof and a compound represented by the following general formula (7) or a salt thereof to obtain a fluorine-containing isoxazolone compound represented by the following general formula (1b): 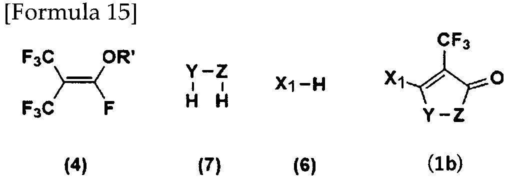 wherein
R' represents a hydrocarbon group having 1 to 12 carbon atoms,
X₁ represents a heterocyclic group, -OA¹, -S(Oₙ)A¹ (where n is an integer of 0 to 2), -O-NA¹A², -NA¹A², -NA³-NA¹A², or -N=A⁴,
A¹, A², A³, and A⁴ each independently represent an organic group having 1 to 12 carbon atoms, and
   (i) Y is NR (where R is an alkyl group having 1 to 12 carbon atoms, an aryl group, an acyl group, an alkoxycarbonyl group, a cycloalkyl group, a carbamoyl group, or -(CH₂)ₐ-B¹, a is an integer of 1 to 12, and B¹ represents an organic group having 1 to 12 carbon atoms), and Z is O, or
   (ii) Y is O, and Z is NR (where R is an alkyl group having 1 to 12 carbon atoms, an aryl group, an acyl group, an alkoxycarbonyl group, a cycloalkyl group, a carbamoyl group, or -(CH₂)ₐ-B¹, a is an integer of 1 to 12, and B¹ represents an organic group having 1 to 12 carbon atoms).
A¹, A², A³, A⁴, R, and R' may be the same groups respectively as A¹, A², A³, A⁴, R, and R' above.

Examples of a salt of the compound represented by the general formula (6) include hydrochlorides, sulfates, and acetates.

Examples of a salt of the compound represented by the general formula (7) include hydrochlorides, sulfates, and acetates.

The step (d) of obtaining a fluorine-containing isoxazolone compound is preferably carried out in the presence of a nucleophilic reagent. Examples of the nucleophilic reagent include 1-methylimidazole, 4-dimethylaminopyridine, 1,4-diazabicyclo[2.2.2]octane, and triphenylphosphine. The use of the nucleophilic reagent can effectively eliminate the substituent -R' from the fluoroisobutene derivative represented by the general formula (4) to promote the generation of the fluorine-containing isoxazolone compound represented by the general formula (1b). Preferably, the step (d) of obtaining a fluorine-containing isoxazolone compound is preferably carried out in the presence of a fluoride ion scavenger. It is preferable to react the fluoroisobutene derivative represented by the general formula (4) with the compound represented by the general formula (6) or a salt thereof and the compound represented by the general formula (7) or a salt thereof in the presence of a fluoride ion scavenger. The fluoride ion scavenger is not particularly limited as long as it is a substance having a function of capturing fluoride ions, and examples thereof include the same fluoride ion scavengers to be used in the step (a) above. It is considered that the cation derived from the fluoride ion scavenger captures fluoride ions liberated from the fluoroisobutane derivative represented by the general formula (4) during the reaction, and precipitates the fluoride ions as a salt having low solubility in an organic solvent to promote the reaction, and thus, the fluorine-containing isoxazolone compound represented by the general formula (1b) can be obtained in a high yield.

The reaction of (d) above, performed in the presence of a nucleophilic reagent, 1-methylimidazole, between the fluoroisobutene derivative represented by the general formula (4) and the compounds represented by the general formulae (6) and (7), is represented by the following reaction formula (D):

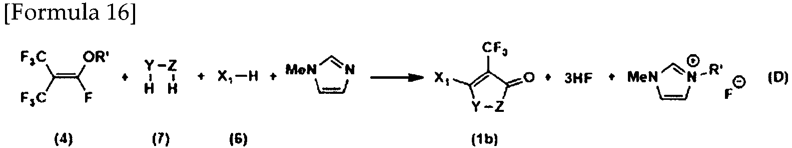

In the method for producing a fluorine-containing isoxazolone compound of this another embodiment, the reaction of (D) above can be carried out in a single step. Consequently, the fluorine-containing isoxazolone compound of the general formula (1b) can be obtained in a simplified manner. In the reaction of (d) above, a cyclic isoxazolone ring structure is formed between the fluoroisobutene derivative represented by the general formula (4) and Y and Z derived from the compound of the general formula (7). Besides, -X₁ and -CF₃ derived from the compound of the general formula (6) and the fluoroisobutene derivative are located at the 5- and 4-positions, respectively, of the 3-isoxazolone ring (in the case of (ii) above in which Y is O and Z is NR), and -X₁ and -CF₃ derived from the compound of the general formula (6) and the fluoroisobutene derivative are located at the 3- and 4-positions, respectively, of the 5-isoxazolone ring (in the case of (i) above in which Y is NR and Z is O).

A reaction temperature during the reaction of (d) above is preferably - 20 to 100°C, more preferably 0 to 50°C, and still more preferably 10 to 30°C. A reaction time during the reaction of (d) above is preferably 0.5 to 48 hours, more preferably 1 to 36 hours, and still more preferably 10 to 25 hours. In the reaction of (d) above, the same hydrogen halide scavenger and solvent as in (a) above can be used.

A method for producing a fluorine-containing isoxazolone compound of another embodiment includes:
(e) a step of reacting a fluoroisobutane derivative represented by the following general formula (5) with a compound represented by the following general formula (7) or a salt thereof to obtain a fluorine-containing isoxazolone compound represented by the following general formula (1a): 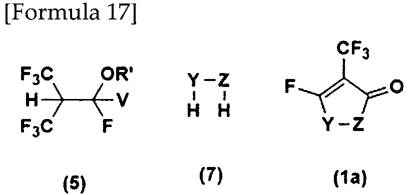 wherein
R' represents a hydrocarbon group having 1 to 12 carbon atoms,
   (i) Y is NR (where R is an alkyl group having 1 to 12 carbon atoms, an aryl group, an acyl group, an alkoxycarbonyl group, a cycloalkyl group, a carbamoyl group, or -(CH₂)ₐ-B¹, a is an integer of 1 to 12, and B¹ represents an organic group having 1 to 12 carbon atoms), and Z is O, or
   (ii) Y is O, and Z is NR (where R is an alkyl group having 1 to 12 carbon atoms, an aryl group, an acyl group, an alkoxycarbonyl group, a cycloalkyl group, a carbamoyl group, or -(CH₂)ₐ-B¹, a is an integer of 1 to 12, and B¹ represents an organic group having 1 to 12 carbon atoms),
V represents a halogen atom, -OA⁵, or -SOₘA⁵ (where m is an integer of 0 to 2), and
A⁵ represents an organic group having 1 to 12 carbon atoms.
R' and R may be the same groups respectively as R' and R above.
V is preferably a halogen atom, and more preferably a fluorine atom.

Examples of A⁵ include a hydrocarbon group and a haloalkyl group, and more specifically include a methyl group, a phenyl group, and a trifluoromethyl group. Examples of a salt of the compound represented by the general formula (7) include hydrochlorides, sulfates, and acetates.

The step (e) of obtaining a fluorine-containing isoxazolone compound is preferably carried out in the presence of a nucleophilic reagent. Examples of the nucleophilic reagent include 1-methylimidazole, 4-dimethylaminopyridine, 1,4-diazabicyclo[2.2.2]octane, and triphenylphosphine. The use of the nucleophilic reagent can effectively eliminate the substituent -R' from the fluoroisobutane derivative represented by the general formula (5) to promote the generation of the fluorine-containing isoxazolone compound represented by the general formula (1a). Preferably, the step (e) of obtaining a fluorine-containing isoxazolone compound is preferably carried out in the presence of a fluoride ion scavenger. It is preferable to react the fluoroisobutane derivative represented by the general formula (5) with the compound represented by the general formula (7) or a salt thereof in the presence of a fluoride ion scavenger. The fluoride ion scavenger is not particularly limited as long as it is a substance having a function of capturing fluoride ions, and examples thereof include the same fluoride ion scavengers to be used in the step (a) above. It is considered that the cation derived from the fluoride ion scavenger captures fluoride ions liberated from the fluoroisobutane derivative represented by the general formula (5) during the reaction, and precipitates the fluoride ions as a salt having low solubility in an organic solvent to promote the reaction, and thus, the fluorine-containing isoxazolone compound represented by the general formula (1a) can be obtained in a high yield.

The reaction of (e) above between the fluoroisobutane derivative represented by the general formula (5) and the compound represented by the general formula (7) is represented by the following reaction formula (E):

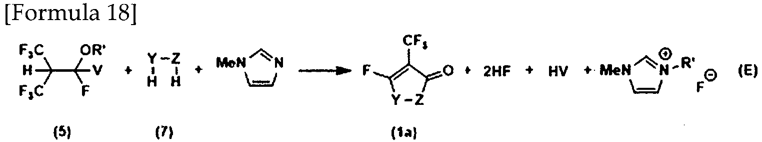

In the method for producing a fluorine-containing isoxazolone compound of this another embodiment, the reaction of (E) above can be carried out in a single step. Consequently, the fluorine-containing isoxazolone compound of the general formula (1a) can be obtained in a simplified manner. In the reaction of (e) above, a cyclic isoxazolone ring structure is formed between the fluoroisobutane derivative represented by the general formula (5) and Y and Z derived from the compound of the general formula (7). Besides, -F and -CF₃ derived from the fluoroisobutane derivative are located at the 5- and 4-positions, respectively, of the 3-isoxazolone ring (in the case of (ii) above in which Y is O and Z is NR), and -F and -CF₃ derived from the fluoroisobutane derivative are located at the 3- and 4-positions, respectively, of the 5-isoxazolone ring (in the case of (i) above in which Y is NR and Z is O).

A reaction temperature during the reaction of (e) above is preferably - 20 to 100°C, more preferably 0 to 50°C, and still more preferably 10 to 30°C. A reaction time during the reaction of (e) above is preferably 0.5 to 48 hours, more preferably 1 to 36 hours, and still more preferably 10 to 25 hours. In the reaction of (e) above, the same hydrogen halide scavenger and solvent as in (a) above can be used.

A method for producing a fluorine-containing isoxazolone compound of another embodiment includes:
(f) a step of reacting a fluoroisobutane derivative represented by the following general formula (5) with a compound represented by the following general formula (6) or a salt thereof and a compound represented by the following general formula (7) or a salt thereof to obtain a fluorine-containing isoxazolone compound represented by the following general formula (1b): 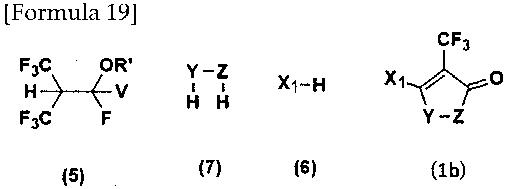 wherein
R' represents a hydrocarbon group having 1 to 12 carbon atoms,
X₁ represents a heterocyclic group, -OA¹, -S(Oₙ)A¹ (where n is an integer of 0 to 2), -O-NA¹A², -NA¹A², -NA³-NA¹A², or -N=A⁴,
A¹, A², A³, and A⁴ each independently represent an organic group having 1 to 12 carbon atoms,
   (i) Y is NR (where R is an alkyl group having 1 to 12 carbon atoms, an aryl group, an acyl group, an alkoxycarbonyl group, a cycloalkyl group, a carbamoyl group, or -(CH₂)ₐ-B¹, a is an integer of 1 to 12, and B¹ represents an organic group having 1 to 12 carbon atoms), and Z is O, or
   (ii) Y is O, and Z is NR (where R is an alkyl group having 1 to 12 carbon atoms, an aryl group, an acyl group, an alkoxycarbonyl group, a cycloalkyl group, a carbamoyl group, or -(CH₂)ₐ-B¹, a is an integer of 1 to 12, and B¹ represents an organic group having 1 to 12 carbon atoms),
V represents a halogen atom, -OA⁵, or -SOₘA⁵ (where m is an integer of 0 to 2), and
A⁵ represents an organic group having 1 to 12 carbon atoms.
A¹, A², A³, A⁴, A⁵, B¹, R', and R may be the same groups respectively as A¹, A², A³, A⁴, A⁵, B¹, R', and R above.

Examples of a salt of the compound represented by the general formula (6) include hydrochlorides, sulfates, and acetates.

Examples of a salt of the compound represented by the general formula (7) include hydrochlorides, sulfates, and acetates.

The step (f) of obtaining a fluorine-containing isoxazolone compound is preferably carried out in the presence of a nucleophilic reagent. Examples of the nucleophilic reagent include 1-methylimidazole, 4-dimethylaminopyridine, 1,4-diazabicyclo[2.2.2]octane, and triphenylphosphine. The use of the nucleophilic reagent can effectively eliminate the substituent -R' from the fluoroisobutane derivative represented by the general formula (5) to promote the generation of the fluorine-containing isoxazolone compound represented by the general formula (1b). Preferably, the step (f) of obtaining a fluorine-containing isoxazolone compound is preferably carried out in the presence of a fluoride ion scavenger. It is preferable to react the fluoroisobutane derivative represented by the general formula (5) with the compound represented by the general formula (6) or a salt thereof, and the compound represented by the general formula (7) or a salt thereof in the presence of a fluoride ion scavenger. The fluoride ion scavenger is not particularly limited as long as it is a substance having a function of capturing fluoride ions, and examples thereof include the same fluoride ion scavengers to be used in the step (a) above. It is considered that the cation derived from the fluoride ion scavenger captures fluoride ions liberated from the fluoroisobutane derivative represented by the general formula (5) during the reaction, and precipitates the fluoride ions as a salt having low solubility in an organic solvent to promote the reaction, and thus, the fluorine-containing isoxazolone compound represented by the general formula (1b) can be obtained in a high yield.

The reaction of (f) above between the fluoroisobutane derivative represented by the general formula (5) and the compounds represented by the general formulae (6) and (7), is represented by the following reaction formula (F):

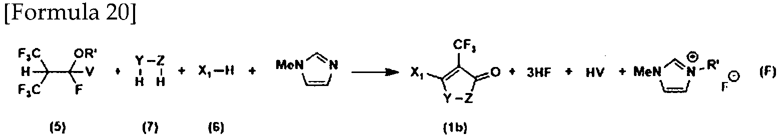

In the method for producing a fluorine-containing isoxazolone compound of this another embodiment, the reaction of (F) above can be carried out in a single step. Consequently, the fluorine-containing isoxazolone compound of the general formula (1b) can be obtained in a simplified manner. In the reaction of (f) above, a cyclic isoxazolone ring structure is formed between the fluoroisobutane derivative represented by the general formula (5) and Y and Z derived from the compound of the general formula (7). Besides, -X₁ and -CF₃ derived from the compound of the general formula (6) and the fluoroisobutane derivative are located at the 5- and 4-positions, respectively, of the 3-isoxazolone ring (in the case of (ii) above in which Y is O and Z is NR), and -X₁ and -CF₃ derived from the compound of the general formula (6) and the fluoroisobutane derivative are located at the 3- and 4-positions, respectively, of the 5-isoxazolone ring (in the case of (i) above in which Y is NR and Z is O).

A reaction temperature during the reaction of (f) above is preferably - 20 to 100°C, more preferably 0 to 50°C, and still more preferably 10 to 30°C. A reaction time during the reaction of (f) above is preferably 0.5 to 48 hours, more preferably 1 to 36 hours, and still more preferably 10 to 25 hours. In the reaction of (f) above, the same hydrogen halide scavenger and solvent as in (a) above can be used.

Although embodiments of the present invention have been described above, the present invention is not limited to the above-described embodiments, and may be modified in various ways within the scope of the present invention, including all aspects included in the concepts and claims of the present invention.

### Examples

To further clarify the effects of the present invention, Examples will be described below, but the present invention is in no way limited to these Examples.

### (Example 1)

### Production of 3-butoxy-2-isopropyl-4-(trifluoromethyl)isoxazol-5-one

Under ice-water cooling, 3 g (24 mmol) of 4-dimethylaminopyridine was dissolved in 30 g of THF. To the resultant solution, 5 g (24 mmol) of 1,3,3,3-tetrafluoro-1-methoxy-2-trifluoromethyl-1-propene was added dropwise such that the internal temperature did not exceed 10°C, and the temperature was raised to room temperature. After about 2 hours, the resulting reaction mixture was added dropwise into 20 g of n-butanol under ice-water cooling such that the internal temperature did not exceed 10°C, and the temperature was raised to room temperature. After about 5 hours, the resulting reaction mixture was cooled with ice water, and 6 g (47 mmol) of diisopropylethylamine was added thereto dropwise such that the internal temperature did not exceed 10°C, and the temperature was raised to room temperature. After about 12 hours, the resulting reaction mixture was cooled with ice water, and a mixed solution of 3 g (24 mmol) of diisopropylethylamine, 2 g (24 mmol) of N-isopropylhydroxylamine, and 5 g of THF was added thereto dropwise such that the internal temperature did not exceed 10°C, and the temperature was raised to room temperature. After about 36 hours, the contents were subjected to silica gel column purification with a mixed solvent of hexane and ethyl acetate in a 7:3 ratio to produce 3-butoxy-2-isopropyl-4-(trifluoromethyl)isoxazol-5-one represented by the following formula (G). The yield of the compound of the formula (G) thus obtained was 0.6 g (isolated yield 9%). In this example, 1,3,3,3-tetrafluoro-1-methoxy-2-trifluoromethyl-1-propene, N-isopropylhydroxylamine, and n-butanol respectively correspond to the fluoroisobutene derivative represented by the general formula (4), the compound of the general formula (7), and the compound of the general formula (6).

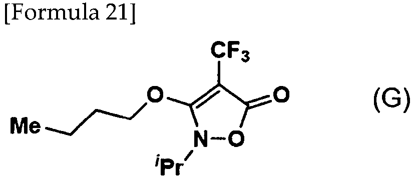

The analysis results of the compound of the formula (G) were as follows.
Mass Spectrum (APCI, m/z): 267 ([M]⁺)

### (Example 2)

### Production of 2-cyclohexyl-3-(ethyl(methoxy)amino)-4-(trifluoromethyl)isoxazol-5-one

Under ice-water cooling, 3 g (34 mmol) of triethylamine was dissolved in 30 g of THF. To the resultant solution, 4 g (17 mmol) of 1,1,1,3,3-pentafluoro-3-methoxy-2-trifluoromethyl-propane was added dropwise such that the internal temperature did not exceed 10°C, and the temperature was raised to room temperature. After about 2 hours, the resulting reaction mixture was added dropwise into 15 g of methanol under ice-water cooling such that the internal temperature did not exceed 10°C, and the temperature was raised to room temperature. After about 5 hours, the resulting reaction mixture was cooled with ice water, 1 g (17 mmol) of N-ethyl-O-methylhydroxylamine was added thereto, and then 3 g (34 mmol) of triethylamine was added thereto dropwise such that the internal temperature did not exceed 10°C, and the temperature was raised to room temperature. After about 12 hours, the resulting reaction mixture was cooled with ice water, and a mixed solution of 2 g (17 mmol) of triethylamine, 2 g (17 mmol) of N-cyclohexylhydroxylamine, and 5 g of THF was added thereto dropwise such that the internal temperature did not exceed 10°C, and the temperature was raised to room temperature. After about 36 hours, the contents were subjected to silica gel column purification with a mixed solvent of hexane and ethyl acetate in a 7:3 ratio to produce 2-cyclohexyl-3-(ethyl(methoxy)amino)-4-(trifluoromethyl)isoxazol-5-one represented by the following formula (H). The yield of the compound of the formula (H) thus obtained was 0.2 g (isolated yield 3%). In this example, 1,1,1,3,3-pentafluoro-3-methoxy-2-trifluoromethyl-propane, N-ethyl-O-methylhydroxylamine, and N-cyclohexylhydroxylamine respectively correspond to the fluoroisobutane derivative of the general formula (5), the compound of the general formula (6), and the compound of the general formula (7).

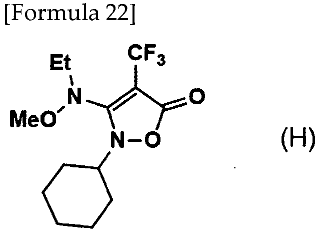

The analysis results of the compound of the formula (H) were as follows.
Mass Spectrum (APCI, m/z): 308 ([M]⁺)

### (Example 3)

### Production of 5-(benzyl(imidazo[1,2-a]pyrimidin-6-yl)amino)-2-methyl-4-(trifluoromethyl)isoxazol-3-one

Under ice-water cooling, 2 g (19 mmol) of N-methylimidazole was dissolved in 30 g of THF. To the resultant solution, 4 g (19 mmol) of 1,3,3,3-tetrafluoro-1-methoxy-2-trifluoromethyl-1-propene was added dropwise such that the internal temperature did not exceed 10°C, and the temperature was raised to room temperature. After about 2 hours, the resulting reaction mixture was cooled with ice water, and a mixed solution of 1 g (19 mmol) of N-methylhydroxylamine and 5 g of THF was added thereto dropwise such that the internal temperature did not exceed 10°C, and the temperature was raised to room temperature. After about 6 hours, the resulting reaction mixture was cooled with ice water, and a mixed solution of 4 g (19 mmol) of N-benzylimidazo[1,2-a]pyrimidin-6-amine, 7 g (57 mmol) of diisopropylethylamine, and 15 g of THF was added thereto dropwise such that the internal temperature did not exceed 10°C, and the temperature was raised to room temperature. After about 48 hours, the contents were subjected to silica gel column purification with a mixed solvent of hexane and ethyl acetate in a 7:3 ratio to produce 5-(benzyl(imidazo[1,2-a]pyrimidin-6-yl)amino)-2-methyl-4-(trifluoromethyl)isoxazol-3-one represented by the following formula (I). The yield of the compound of the formula (I) thus obtained was 0.1 g (isolated yield 2%). In this example, 1,3,3,3-tetrafluoro-1-methoxy-2-trifluoromethyl-1-propene, N-methylhydroxylamine, and N-benzylimidazo[1,2-a]pyrimidin-6-amine correspond respectively to the fluoroisobutene derivative of the general formula (4), the compound of the general formula (7), and the compound of the general formula (6).

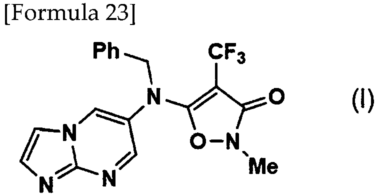

The analysis results of the compound of the formula (I) were as follows.
Mass Spectrum (APCI, m/z): 389 ([M]⁺)

### (Example 4)

### Production of 1,1-dimethylethyl N-[3-({2-[(4-methoxyphenyl)methyl]-5-(trifluoromethyl)-3-oxo-5-isoxazolyl}methylamino)propyl]carbamate

0.3 g (3.2 mmol) of 1-methylimidazole was dissolved in 3.0 ml of THF, and after cooling the resultant to 0°C, 0.6 g (2.9 mmol) of 1,3,3,3-tetrafluoro-1-methoxy-2-trifluoromethyl-1-propene was added thereto, followed by stirring for 19.3 hours at room temperature. After cooling the resultant to 0°C, 0.5 g (3.0 mmol) of N-(4-methoxybenzyl)hydroxylamine was added thereto, and after stirring the resultant for 7.4 hours at room temperature, the resulting mixture was cooled to 0°C. Then, 0.5 g (2.8 mmol) of tert-butyl [3-(methylamino)propyl]carbamate and 0.7 g (5.7 mmol) of diisopropylethylamine were added thereto, followed by stirring the resultant for 64.4 hours at room temperature to produce 1,1-dimethylethyl N-[3-({2-[(4-methoxyphenyl)methyl]-5-(trifluoromethyl)-3-oxo-5-isoxazolyl}methylamino)propyl]carbamate represented by the following formula (J). In this example, 1,3,3,3-tetrafluoro-1-methoxy-2-trifluoromethyl-1-propene, N-(4-methoxybenzyl)hydroxylamine, and tert-butyl [3-(methylamino)propyl]carbamate respectively correspond to the fluoroisobutene derivative of the general formula (4), the compound of the general formula (7), and the compound of the general formula (6).

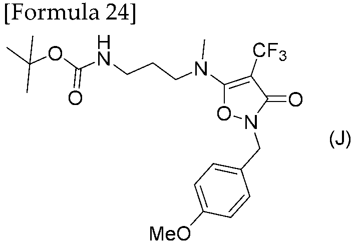

The analysis results of the compound of the formula (J) were as follows.
Mass Spectrum (APCI, m/z): 458.9 ([M]⁺)

### (Example 5)

### Production of 2-hexyl-3-[methoxy(phenylmethyl)amino]-4-(trifluoromethyl)isoxazol-5-one

Under ice-water cooling, 0.8 g (7.6 mmol) of 4-methylmorpholine was dissolved in 5.4 ml of THF. To the resultant solution, 0.9 g (3.8 mmol) of 1,1,1,3,3-pentafluoro-3-methoxy-2-trifluoromethyl-propane was added dropwise such that the internal temperature did not exceed 10°C, and the temperature was raised to room temperature. After about 2 hours, the resulting reaction mixture was added dropwise into 10.8 ml of methanol under ice-water cooling such that the internal temperature did not exceed 10°C, and the temperature was raised to room temperature. After about 5 hours, the resulting reaction mixture was cooled with ice water, then 0.5 g (3.7 mmol) of N-benzyl-O-methylhydroxylamine was added thereto, and 1.0 g (7.4 mmol) of N,N-diisopropylethylamine was added thereto dropwise such that the internal temperature did not exceed 10°C, and the temperature was raised to room temperature. After about 19 hours, the resulting reaction mixture was cooled with ice water, and a mixed solution of 1.0 g (7.3 mmol) of N,N-diisopropylethylamine, 0.6 g (3.7 mmol) of N-hexylhydroxylamine hydrochloride, and 1 ml of THF was added thereto dropwise such that the internal temperature did not exceed 10°C, and the temperature was raised to room temperature. After about 25 hours, the contents were subjected to silica gel column purification to produce 2-hexyl-3-[methoxy(phenylmethyl)amino]-4-(trifluoromethyl)isoxazol-5-one represented by the following formula (K). In this example, 1,1,1,3,3-pentafluoro-3-methoxy-2-trifluoromethyl-propane, N-benzyl-O-methylhydroxylamine, and N-hexylhydroxylamine hydrochloride respectively correspond to the fluoroisobutane derivative of the general formula (5), the compound of the general formula (6), and the salt of the compound of the general formula (7).

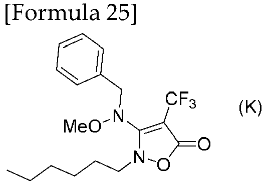

The analysis results of the compound of the formula (K) were as follows.
Mass Spectrum (APCI, m/z): 372.0 ([M]⁺)

### (Example 6)

### Production of 3-[(2-methoxyethyl)propylamino]-2-(2-thienylmethyl)-4-(trifluoromethyl)isoxazol-5-one

Under ice-water cooling, 0.9 g (8.8 mmol) of triethylamine was dissolved in 15 ml of THF. To the resultant solution, 1.0 g (4.4 mmol) of 1,1,1,3,3-pentafluoro-3-methoxy-2-trifluoromethyl-propane was added dropwise such that the internal temperature did not exceed 10°C, and the temperature was raised to room temperature. After about 2 hours, the resulting reaction mixture was added dropwise into 30 ml of methanol under ice-water cooling such that the internal temperature did not exceed 10°C, and the temperature was raised to room temperature. After about 5 hours, the resulting reaction mixture was cooled with ice water, then 0.5 g (4.5 mmol) of N-(2-methoxyethyl)propylamine was added thereto, and 1.1 g (8.6 mmol) of N,N-diisopropylethylamine was added thereto dropwise such that the internal temperature did not exceed 10°C, and the temperature was raised to room temperature. After about 23 hours, the resulting reaction mixture was cooled with ice water, and a mixed solution of 1.1 g (8.5 mmol) of N,N-diisopropylethylamine, 0.6 g (4.3 mmol) of N-[(thiophen-2-yl)methyl]hydroxylamine, and 2 ml of THF was added thereto dropwise such that the internal temperature did not exceed 10°C, and the temperature was raised to room temperature. After about 30 hours, the contents were subjected to silica gel column purification to produce 3-[(2-methoxyethyl)propylamino]-2-(2-thienylmethyl)-4-(trifluoromethyl)isoxazol-5-one represented by the following formula (L). In this example, 1,1,1,3,3-pentafluoro-3-methoxy-2-trifluoromethyl-propane, N-(2-methoxyethyl)propylamine, and N-[(thiophen-2-yl)methyl]hydroxylamine respectively correspond to the fluoroisobutane derivative of the general formula (5), the compound of the general formula (6), and the compound of the general formula (7).

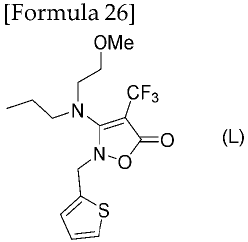

The analysis results of the compound of the formula (L) were as follows.
Mass Spectrum (APCI, m/z): 364.0 ([M]⁺)

### (Example 7)

### Production of 2-[(4-methoxyphenyl)methyl]-3-[methyl(2-thienylmethyl)amino]-4-(trifluoromethyl)isoxazol-5-one

Under ice-water cooling, 0.9 g (6.6 mmol) of N,N-dimethylhexylamine was dissolved in 5 ml of THF. To the resultant solution, 0.8 g (3.3 mmol) of 1,1,1,3,3-pentafluoro-3-methoxy-2-trifluoromethyl-propane was added dropwise such that the internal temperature did not exceed 10°C, and the temperature was raised to room temperature. After about 2 hours, the resulting reaction mixture was added dropwise into 10 ml of methanol under ice-water cooling such that the internal temperature did not exceed 10°C, and the temperature was raised to room temperature. After about 5 hours, the resulting reaction mixture was cooled with ice water, then 0.4 g (3.2 mmol) of N-methyl-1-(thiophen-2-yl)methanamine was added thereto, and 0.8 g (6.4 mmol) of N,N-diisopropylethylamine was added thereto dropwise such that the internal temperature did not exceed 10°C, and the temperature was raised to room temperature. After about 23 hours, the resulting reaction mixture was cooled with ice water, and a mixed solution of 0.4 g (3.3 mmol) of N,N-diisopropylethylamine, 0.5 g (3.1 mmol) of N-(4-methoxybenzyl)hydroxylamine, and 2 ml of THF was added thereto dropwise such that the internal temperature did not exceed 10°C, and the temperature was raised to room temperature. After about 24 hours, the contents were subjected to silica gel column purification to produce 2-[(4-methoxyphenyl)methyl]-3-[methyl(2-thienylmethyl)amino]-4-(trifluoromethyl)isoxazol-5-one represented by the following formula (M). In this example, 1,1,1,3,3-pentafluoro-3-methoxy-2-trifluoromethyl-propane, N-methyl-1-(thiophen-2-yl)methanamine, and N-(4-methoxybenzyl)hydroxylamine respectively correspond to the fluoroisobutane derivative of the general formula (5), the compound of the general formula (6), and the compound of the general formula (7).

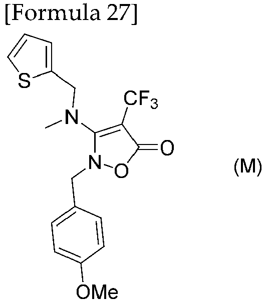

The analysis results of the compound of the formula (M) were as follows.
Mass Spectrum (APCI, m/z): 398.0 ([M]⁺)

### (Example 8)

### Production of 3-(2-isoxazolidinyl)-2-(4-phenylbutyl)-4-(trifluoromethyl)isoxazol-5-one

Under ice-water cooling, 1.2 g (9.2 mmol) of N,N-dimethylbenzylamine was dissolved in 6.7 ml of THF. To the resultant solution, 1.1 g (4.6 mmol) of 1,1,1,3,3-pentafluoro-3-methoxy-2-trifluoromethyl-propane was added dropwise such that the internal temperature did not exceed 10°C, and the temperature was raised to room temperature. After about 2 hours, the resulting reaction mixture was added dropwise into 13.4 ml of methanol under ice-water cooling such that the internal temperature did not exceed 10°C, and the temperature was raised to room temperature. After about 5 hours, the resulting reaction mixture was cooled with ice water, then 0.5 g (4.7 mmol) of isoxazolidine hydrochloride was added thereto, and 1.8 g (13.9 mmol) of N,N-diisopropylethylamine was added thereto dropwise such that the internal temperature did not exceed 10°C, and the temperature was raised to room temperature. After about 19 hours, the resulting reaction mixture was cooled with ice water, and a mixed solution of 1.2 g (9.1 mmol) of N,N-diisopropylethylamine, 0.9 g (4.6 mmol) of N-(4-phenylbutyl)hydroxylamine hydrochloride, and 2 ml of THF was added thereto dropwise such that the internal temperature did not exceed 10°C, and the temperature was raised to room temperature. After about 23 hours, the contents were subjected to silica gel column purification to produce 3-(2-isoxazolidinyl)-2-(4-phenylbutyl)-4-(trifluoromethyl)isoxazol-5-one represented by the following formula (N). In this example, 1,1,1,3,3-pentafluoro-3-methoxy-2-trifluoromethyl-propane, isoxazolidine hydrochloride, and N-(4-phenylbutyl)hydroxylamine hydrochloride respectively correspond to the fluoroisobutane derivative of the general formula (5), a salt of the compound of the general formula (6), and a salt of the compound of the general formula (7).

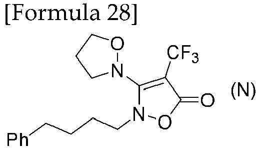

The analysis results of the compound of the formula (N) were as follows.
Mass Spectrum (APCI, m/z): 356.3 ([M]⁺)

### (Example 9)

### Production of 1,1-dimethylethyl-N-[2-({2-butyl-3-(trifluoromethyl)-5-oxo-3-isoxazolyl}methylamino)ethyl]carbamate

Under ice-water cooling, 0.8 g (6.0 mmol) of N,N-dimethylbenzylamine was dissolved in 4.2 ml of THF. To the resultant solution, 0.7 g (3.0 mmol) of 1,1,1,3,3-pentafluoro-3-methoxy-2-trifluoromethyl-propane was added dropwise such that the internal temperature did not exceed 10°C, and the temperature was raised to room temperature. After about 2 hours, the resulting reaction mixture was added dropwise into 8.4 ml of methanol under ice-water cooling such that the internal temperature did not exceed 10°C, and the temperature was raised to room temperature. After about 5 hours, the resulting reaction mixture was cooled with ice water, then 0.5 g (2.8 mmol) of N-(tert-butoxycarbonyl)-N'-methylethylenediamine was added thereto, and 0.8 g (5.8 mmol) of N,N-diisopropylethylamine was added thereto dropwise such that the internal temperature did not exceed 10°C, and the temperature was raised to room temperature. After about 20 hours, the resulting reaction mixture was cooled with ice water, and a mixed solution of 0.8 g (5.9 mmol) of N,N-diisopropylethylamine, 0.4 g (2.9 mmol) of N-butylhydroxylamine hydrochloride, and 2 ml of THF was added thereto dropwise such that the internal temperature did not exceed 10°C, and the temperature was raised to room temperature. After about 30 hours, the contents were subjected to silica gel column purification to produce 1,1-dimethylethyl-N-[2-({2-butyl-3-(trifluoromethyl)-5-oxo-3-isoxazolyl}methylamino)ethyl]carbamate represented by the following formula (O). In this example, 1,1,1,3,3-pentafluoro-3-methoxy-2-trifluoromethyl-propane, N-(tert-butoxycarbonyl)-N'-methylethylenediamine, and N-butylhydroxylamine hydrochloride respectively correspond to the fluoroisobutane derivative of the general formula (5), the compound of the general formula (6), and a salt of the compound of the general formula (7).

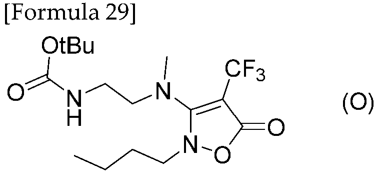

The analysis results of the compound of the formula (O) were as follows.
Mass Spectrum (APCI, m/z): 381.2 ([M]⁺)

### (Example 10)

### Production of 3-{[1-(1,1-dimethylethyl)-2,2-dimethylpropylidine]amino}-2-(phenylmethyl)-4-(trifluoromethyl)isoxazol-5-one

Under ice-water cooling, 1.5 g (14.4 mmol) of triethylamine was dissolved in 10.3 ml of THF. To the resultant solution, 1.7 g (7.2 mmol) of 1,1,1,3,3-pentafluoro-3-methoxy-2-trifluoromethyl-propane was added dropwise such that the internal temperature did not exceed 10°C, and the temperature was raised to room temperature. After about 2 hours, the resulting reaction mixture was added dropwise into 20.6 ml of methanol under ice-water cooling such that the internal temperature did not exceed 10°C, and the temperature was raised to room temperature. After about 5 hours, the resulting reaction mixture was cooled with ice water, then 1.0 g (7.2 mmol) of 2,2,4,4-tetramethyl-3-pentanonimine was added thereto, and 1.9 g (14.4 mmol) of N,N-diisopropylethylamine was added thereto dropwise such that the internal temperature did not exceed 10°C, and the temperature was raised to room temperature. After about 17 hours, the resulting reaction mixture was cooled with ice water, and a mixed solution of 1.8 g (14.2 mmol) of N,N-diisopropylethylamine, 1.1 g (7.1 mmol) of N-benzylhydroxylamine hydrochloride, and 2 ml of THF was added thereto dropwise such that the internal temperature did not exceed 10°C, and the temperature was raised to room temperature. After about 71 hours, the contents were subjected to silica gel column purification to produce 3-{[1-(1,1-dimethylethyl)-2,2-dimethylpropylidine]amino}-2-(phenylmethyl)-4-(trifluoromethyl)isoxazol-5-one represented by the following formula (P). In this example, 1,1,1,3,3-pentafluoro-3-methoxy-2-trifluoromethyl-propane, 2,2,4,4-tetramethyl-3-pentanonimine, and N-benzylhydroxylamine hydrochloride respectively correspond to the fluoroisobutane derivative of the general formula (5), the compound of the general formula (6), and a salt of the compound of the general formula (7).

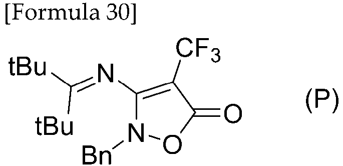

The analysis results of the compound of the formula (P) were as follows.
Mass Spectrum (APCI, m/z): 382.4 ([M]⁺)

## Claims

1. A fluorine-containing isoxazolone compound represented by the following general formula (1): 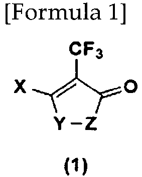 wherein
X represents a fluorine atom, a heterocyclic group, -OA¹, -S(Oₙ)A¹, where n is an integer of 0 to 2, -O-NA¹A², -NA¹A², -NA³-NA¹A², or -N=A⁴,
A¹, A², A³, and A⁴ each independently represent an organic group having 1 to 12 carbon atoms, and
(i) Y is NR, where R is an alkyl group having 1 to 12 carbon atoms, an aryl group, an acyl group, an alkoxycarbonyl group, a cycloalkyl group, a carbamoyl group, or -(CH₂)ₐ-B¹, a is an integer of 1 to 12, and B¹ represents an organic group having 1 to 12 carbon atoms, and Z is O, or
(ii) Y is O, and Z is NR, where R is an alkyl group having 1 to 12 carbon atoms, an aryl group, an acyl group, an alkoxycarbonyl group, a cycloalkyl group, a carbamoyl group, or -(CH₂)ₐ-B¹, a is an integer of 1 to 12, and B¹ represents an organic group having 1 to 12 carbon atoms.

2. A method for producing a fluorine-containing isoxazolone compound, comprising a step of reacting a fluoroisobutanoic acid fluoride derivative represented by the following general formula (2) with a compound represented by the following general formula (7) or a salt thereof to obtain a fluorine-containing isoxazolone compound represented by the following general formula (1a): 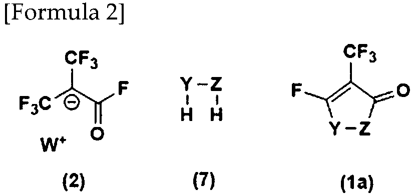 wherein
(i) Y is NR, where R is an alkyl group having 1 to 12 carbon atoms, an aryl group, an acyl group, an alkoxycarbonyl group, a cycloalkyl group, a carbamoyl group, or -(CH₂)ₐ-B¹, a is an integer of 1 to 12, and B¹ represents an organic group having 1 to 12 carbon atoms, and Z is O, or
(ii) Y is O, and Z is NR, where R is an alkyl group having 1 to 12 carbon atoms, an aryl group, an acyl group, an alkoxycarbonyl group, a cycloalkyl group, a carbamoyl group, or -(CH₂)ₐ-B¹, a is an integer of 1 to 12, and B¹ represents an organic group having 1 to 12 carbon atoms, and
W⁺ represents an ammonium cation, an imidazolium cation, a pyridinium cation, or a phosphonium cation.

3. A method for producing a fluorine-containing isoxazolone compound, comprising a step of reacting a fluoroisobutanoic acid fluoride derivative represented by the following general formula (2) with a compound represented by the following general formula (6) or a salt thereof and a compound represented by the following general formula (7) or a salt thereof to obtain a fluorine-containing isoxazolone compound represented by the following general formula (1b): 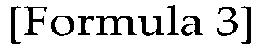 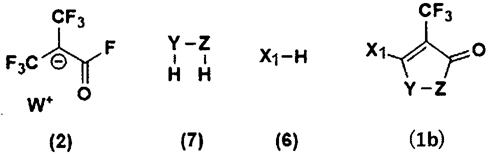 wherein
X₁ represents a heterocyclic group, -OA¹, -S(Oₙ)A¹, where n is an integer of 0 to 2, -O-NA¹A², -NA¹A², -NA³-NA¹A², or -N=A⁴,
A¹, A², A³, and A⁴ each independently represent an organic group having 1 to 12 carbon atoms,
(i) Y is NR, where R is an alkyl group having 1 to 12 carbon atoms, an aryl group, an acyl group, an alkoxycarbonyl group, a cycloalkyl group, a carbamoyl group, or -(CH₂)ₐ-B¹, a is an integer of 1 to 12, and B¹ represents an organic group having 1 to 12 carbon atoms, and Z is O, or
(ii) Y is O, and Z is NR, where R is an alkyl group having 1 to 12 carbon atoms, an aryl group, an acyl group, an alkoxycarbonyl group, a cycloalkyl group, a carbamoyl group, or -(CH₂)ₐ-B¹, a is an integer of 1 to 12, and B¹ represents an organic group having 1 to 12 carbon atoms, and
W⁺ represents an ammonium cation, an imidazolium cation, a pyridinium cation, or a phosphonium cation.

4. A method for producing a fluorine-containing isoxazolone compound, comprising a step of reacting a fluoroisobutene derivative represented by the following general formula (4) with a compound represented by the following general formula (7) or a salt thereof to obtain a fluorine-containing isoxazolone compound represented by the following general formula (1a): 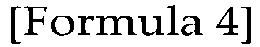 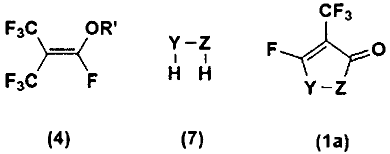 wherein
R' represents a hydrocarbon group having 1 to 12 carbon atoms, and
(i) Y is NR, where R is an alkyl group having 1 to 12 carbon atoms, an aryl group, an acyl group, an alkoxycarbonyl group, a cycloalkyl group, a carbamoyl group, or -(CH₂)ₐ-B¹, a is an integer of 1 to 12, and B¹ represents an organic group having 1 to 12 carbon atoms, and Z is O, or
(ii) Y is O, and Z is NR, where R is an alkyl group having 1 to 12 carbon atoms, an aryl group, an acyl group, an alkoxycarbonyl group, a cycloalkyl group, a carbamoyl group, or -(CH₂)ₐ-B¹, a is an integer of 1 to 12, and B¹ represents an organic group having 1 to 12 carbon atoms.

5. A method for producing a fluorine-containing isoxazolone compound, comprising a step of reacting a fluoroisobutene derivative represented by the following general formula (4) with a compound represented by the following general formula (6) or a salt thereof and a compound represented by the following general formula (7) or a salt thereof to obtain a fluorine-containing isoxazolone compound represented by the following general formula (1b): 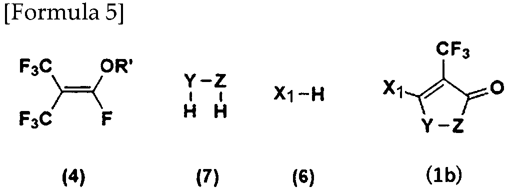 wherein
R' represents a hydrocarbon group having 1 to 12 carbon atoms,
X₁ represents a heterocyclic group, -OA¹, -S(Oₙ)A¹, where n is an integer of 0 to 2, -O-NA¹A², -NA¹A², -NA³-NA¹A², or -N=A⁴,
A¹, A², A³, and A⁴ each independently represent an organic group having 1 to 12 carbon atoms, and
(i) Y is NR, where R is an alkyl group having 1 to 12 carbon atoms, an aryl group, an acyl group, an alkoxycarbonyl group, a cycloalkyl group, a carbamoyl group, or -(CH₂)ₐ-B¹, a is an integer of 1 to 12, and B¹ represents an organic group having 1 to 12 carbon atoms, and Z is O, or
(ii) Y is O, and Z is NR, where R is an alkyl group having 1 to 12 carbon atoms, an aryl group, an acyl group, an alkoxycarbonyl group, a cycloalkyl group, a carbamoyl group, or -(CH₂)ₐ-B¹, a is an integer of 1 to 12, and B¹ represents an organic group having 1 to 12 carbon atoms.

6. A method for producing a fluorine-containing isoxazolone compound, comprising a step of reacting a fluoroisobutane derivative represented by the following general formula (5) with a compound represented by the following general formula (7) or a salt thereof to obtain a fluorine-containing isoxazolone compound represented by the following general formula (1a): 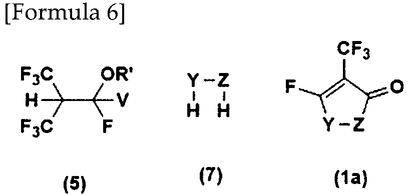 wherein
R' represents a hydrocarbon group having 1 to 12 carbon atoms,
(i) Y is NR, where R is an alkyl group having 1 to 12 carbon atoms, an aryl group, an acyl group, an alkoxycarbonyl group, a cycloalkyl group, a carbamoyl group, or -(CH₂)ₐ-B¹, a is an integer of 1 to 12, and B¹ represents an organic group having 1 to 12 carbon atoms, and Z is O, or
(ii) Y is O, and Z is NR, where R is an alkyl group having 1 to 12 carbon atoms, an aryl group, an acyl group, an alkoxycarbonyl group, a cycloalkyl group, a carbamoyl group, or -(CH₂)ₐ-B¹, a is an integer of 1 to 12, and B¹ represents an organic group having 1 to 12 carbon atoms,
V represents a halogen atom, -OA⁵, or -SOₘA⁵, where m is an integer of 0 to 2, and
A⁵ represents an organic group having 1 to 12 carbon atoms.

7. A method for producing a fluorine-containing isoxazolone compound, comprising a step of reacting a fluoroisobutane derivative represented by the following general formula (5) with a compound represented by the following general formula (6) or a salt thereof and a compound represented by the following general formula (7) or a salt thereof to obtain a fluorine-containing isoxazolone compound represented by the following general formula (1b): 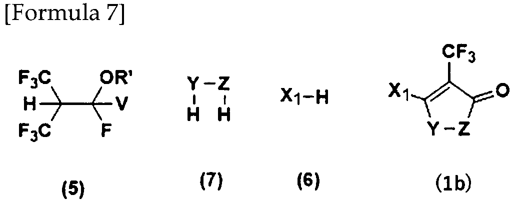 wherein
R' represents a hydrocarbon group having 1 to 12 carbon atoms,
X₁ represents a heterocyclic group, -OA¹, -S(Oₙ)A¹, where n is an integer of 0 to 2, -O-NA¹A², -NA¹A², -NA³-NA¹A², or -N=A⁴,
A¹, A², A³, and A⁴ each independently represent an organic group having 1 to 12 carbon atoms,
(i) Y is NR, where R is an alkyl group having 1 to 12 carbon atoms, an aryl group, an acyl group, an alkoxycarbonyl group, a cycloalkyl group, a carbamoyl group, or -(CH₂)ₐ-B¹, a is an integer of 1 to 12, and B¹ represents an organic group having 1 to 12 carbon atoms, and Z is O, or
(ii) Y is O, and Z is NR, where R is an alkyl group having 1 to 12 carbon atoms, an aryl group, an acyl group, an alkoxycarbonyl group, a cycloalkyl group, a carbamoyl group, or -(CH₂)ₐ-B¹, a is an integer of 1 to 12, and B¹ represents an organic group having 1 to 12 carbon atoms,
V represents a halogen atom, -OA⁵, or -SOₘA⁵, where m is an integer of 0 to 2, and
A⁵ represents an organic group having 1 to 12 carbon atoms.
